# NEW EUROPEAN PATENT SPECIFICATION

(11) **EP 1 494 543 B2**
(45) Date of publication and mention of the opposition decision: **30.12.2020**
(45) Mention of the grant of the patent: 12.11.2014
(21) Application number: 03718392.8
(22) Date of filing: 14.04.2003
(51) Int. Cl.: A23K 1/16, A23K 1/18

(54) **DIETARY METHODS FOR CANINE PERFORMANCE ENHANCEMENT**
DIÄTETISCHE ANWENDUNG IN VERFAHREN ZUR VERBESSERUNG DER LEISTUNG VON HUNDEN
PROCÉDÉS ALIMENTAIRES POUR AMÉLIORER LES PERFORMANCES CANINES

(30) Priority: 12.04.2002 US 121325
(43) Date of publication of application: 12.01.2005
(73) Proprietor: Tetra GmbH, 49324 Melle (DE)
(72) Inventor: DAVENPORT, Gary, Mitchell, Dayton, Ohio 45415 (US); KELLY, Russell, Lee, Eaton, Ohio 45320 (US); ALTOM, Eric, Karl, Eaton, Ohio 45320 (US); LEPINE, Allan, John, Dayton, Ohio 45414 (US)
(74) Representative: Schiweck Weinzierl Koch Patentanwälte Partnerschaft mbB
(86) International application number: PCT/US2003/011509
(87) International publication number: WO 2003/086100

(56) References cited:
- EP-A- 0 678 247
- WO-A-01/37678
- US-A- 6 156 355
- US-B1- 6 297 280
- GARY M. DAVENPORT ET AL.: "Effect of diet on hunting performance of English pointers" VETERINARY THERAPEUTICS, vol. 2, no. 1, 2001, pages 10-23, XP001161257 cited in the application
- SIMOPOULOS, A.P: "The return of omega-3 fatty acids into the food supply. I. Land-based animal food products and their health effects" 1998 , S. KARGER , BASEL XP002172305 Part 3: Companion Animal Nutrition. "Utilization of omega-3 fatty acids in companion animal nutrition", pages 176-185
- HANSEN R A ET AL: "Duration of effects of dietary fish oil supplementation on serum eicosapentaenoic acid and docosahexaenoic acid concentrations in dogs" AMERICAN JOURNAL OF VETERINARY RESEARCH, XX, XX, vol. 59, no. 7, July 1998 (1998-07), pages 864-888, XP000990456 ISSN: 0002-9645
- GILETTE: http://www.sportsvet.com

## Description

### Background of the Invention

The present invention generally relates to diet based methods for maintaining the body temperature of a hunting mammal.

Many canine food formulations on the market are so-called maintenance diets which are designed and formulated to provide adequate nutrition for the average companion dog on a day-to-day basis. There are also special-purpose diets designed to meet special needs or specific nutritional requirements, such as those encountered in pregnancy, nursing, high stress periods, or growth. A number of scientifically designed special-purpose diet formulas are commercially available which can provide desired outcomes in an animal, for example, growth, maintenance, or high energy. The amount of food an animal requires for maintenance or growth depends upon, for example, age, activity, and environment.

An important objective of animal owners, trainers, or care givers is to provide their animal with a diet which maintains overall health, well being, appearance, and prevents disease of the animal; all at a reasonable cost. Another objective is to provide a diet which promotes optimum performance in specific tasks, for example, high energy expenditure activities associated with show, sport, or work animals. Performance diets are known in the art and typically contain, for example, about 25-35 % by weight protein and 18-22 % by weight fat, and can be used mainly for working or show dogs, pregnant or nursing bitches, or animals under stress. However, performance diets are not recommended for all pets, particularly those with a sedentary lifestyle unless food allowances are controlled.

Hunting with dogs for sport or in competitive events is a popular pastime in the United States and elsewhere around the world. The breed or type of dog that is used varies with the type of game that is hunted, as well as the terrain and distances that are covered. Regardless of these differences, most hunting involves several hours of endurance activity interrupted by short periods of intense running or sprinting. If a dog is hunted often during a season, major nutritional concerns are feeding to promote optimal performance and providing enough calories to maintain body weight and body condition. As a result, nutritional programs for these canine athletes must be designed to provide ample energy and other nutrients to support muscle contraction during athletic events while allowing the dog to benefit from training over the course of the season. Both the immediate needs of the muscle as well as the longer-term concerns such as aerobic capacity, proneness to injury and blood volume must be met with the proper nutritional strategy. (Reinhart, G.A., Nutrition for Sporting Dogs. In: Canine Sports Medicine and Surgery, Bloomberg, M.S., Dee, J.F., Taylor, R.A., eds., Philadelphia, 1998, W.B. Saunders, pp 349.) The nutrient needs of canine athletes have received increased attention in recent years, resulting in improved understanding of the changes that are associated with athletic conditioning in this species. The majority of published reports have studied racing greyhounds, endurance sled dogs, or dogs exercising on treadmills in a laboratory setting. (Kronfeld, D.S., Diet and the Performance of Racing Sled Dogs, J. Am. Vet. Med. Assoc., 162:470B473, 1973; Hammel, E.P., et al., Metabolic Responses To Exhaustive Exercise in Racing Sled Dogs Fed Diets Containing Medium, Low and Zero Carbohydrate, Am. J. Clin. Nutr., 1976; 30:409-418; Rose, R.J., et al., Responses to Sprint Exercise in the Greyhound: Effects on Hematology, Serum Biochemistry and Muscle Metabolites, Res. Vet. Sci. 1989, 47:212-218; Toll, P.W., et al., The Effect of Dietary Fat and Carbohydrate on Sprint Performance in Racing Greyhound Dogs, In: Proc 8th Internat. Racing Greyhound Symp. 1992, Gainesville, FLA, pp. 1-3.) Much less is known about the nutritional needs of other types of working dogs. These include, but are not limited to, dogs that are used for hunting, herding, obedience competitions, agility events, and tracking tests, as well as dogs trained to assist the disabled. For each of these categories, the intensity of training and amount of physical work that the dog is required to do can vary considerably. Nevertheless, working dogs typically have increased energy needs compared with the maintenance requirements of normal, adult dogs. An issue is the magnitude of this increase, as well as the best way to supply both energy and other essential nutrients to these working dogs to support maximum performance and well-being.

Dietary methods for improving mammalian energetic performance are known, reference for example the following patent.

In U.S. Pat. No. 6,159,942, issued December 12, 2000, to St. Cyr, et al., there is disclosed a method whereby precursors of adenosine triphosphate are administered orally to increase intracellular ATP concentration as dietary supplements or for treatment of reduced energy availability resulting from strenuous physical activity, illness or trauma. Pentose sugars are administered individually, mixed into dry food or in solution. The preferred pentose is D-ribose, singly or combined with creatine, pyruvate, L-carnitine and/or vasodilating agents. Additionally, magnesium, electrolytes, fatty acids and hexose sugars can be used. The compositions and methods of this invention are especially beneficial to mammals having reduced energy availability or high energy demand.

In U.S. Pat. No. 6,156,355, issued December 5, 2000, to Shields, Jr., et al., there is disclosed breed-specific dog food formulations that comprise chicken meat as the major ingredient, rice as the predominant (or sole) grain source, fruit and/or vegetable fiber as the primary or sole fiber source, unique fat and antioxidant blend, vitamins, herbs and spices, carotenoids, and no corn or artificial colors, preservatives, flavors or sugars.

Other patents related to the formulation of animal diets include U.S. Patent Nos. 5,616,569 (fermentable fiber), 5,932,258 (glucose metabolism) and 6,133,323 (beta-carotene).

The effects of diet on physical performance and olfaction have been noted, see Altom, E., "Effect of Dietary Fat and Physical Conditioning on the Metabolic and Physiological Responses of the Canine Athlete," Ph.D. Dissertation, Auburn University, Auburn, Alabama, 168 pages, March 19,1999.

Certain unsaturated fatty acids, such as the essential fatty acids, are known to play a role in, for example, neural development (see Nutritional Reviews, Vol. 59, No. 8, S34, Aug 2001), retinal composition (see Lipids, Vol. 33, No. 12,1187, 1998), and inflammation response (see Nutrition, Vol. 16, No. 11/12, 1116, 2000).

There exists a need for simple, nutritional based methods which maintain the body temperature of hunting mammals, that is, mammals that are involved in regular or highly energetic physical activity, for example, a trained dog participating in hunt or search activities. A need also exists for a method to increase the energy level of mammals to provide an increased feeling of well-being, alertness, and lower body temperature during periods of high physical activity and caloric expenditure.

### Summary of the Invention

The invention is defined as in the appended claims.

### Detailed Description

The following definitions are used, unless otherwise described.

A "target find" or "point" in the context of bird-dog hunting is the number of times the bird-dog pointed to and indicated the presence of one or more birds in a specific direction or location during a hunt session. A "target find" or "point" in the context of a search animal is, for example, the number of times the search animal pointed to and indicated the presence of one or more target items or search objects in a specific direction or location during a search session.

"Hunt performance" is the number of target finds per hunt.

"Overall hunt performance" is a particular animal's total number of "target finds" or "points" per total hours hunted during a hunting season.

"Target," "target item," or "target object" can mean, for example, a game animal, prey animal, or search object.

"Search performance" or "detection performance" is the number of target finds per total available targets in a search assignment or detection detail. Thus, for example, a search dog finding 8 of 10 known avalanche victims would have a search performance or detection performance of four-fifths or 80 percent.

"Search performance", "detection performance", and "hunt performance" are to be construed broadly. In the context of the present invention these terms can be synonymous and interchangeable.

"Game animal" or "prey animal" are both broadly construed and include any animal, living or otherwise, which is a desired object of a hunter or hunt animal, and can include, for example, wild or domestic animals, small game, such as squirrel, rabbit, and the like, or larger game, such as fox, boar, deer, bear, buffalo, and like animals. Other common prey animals include, but are not limited to, ground-dwelling or game birds, such as, grouse, turkey, pheasant, quail, dove, partridge, and water fowl, such as ducks and geese, and other common fowl.

"Search object" is also construed broadly and can include any living or non-living item, natural or unnatural, which is desired by the searcher or search animal and which search object is to be, for example, discovered, detected, tracked, retrieved, flushed, corralled, and like activities. Examples of search objects include but are not limited to contraband such as narcotics, explosives, herd animals, avalanche or earthquake victims, missing persons, suspects, arrestees, prisoners, prison escapees, trespassers, illegal aliens, terrorists, and the like.

"Mammal" can include, for example, wild or domestic animals, such as dogs, cats, ferrets, and like animals.

The terms "include", "for example", "such as", and the like are used illustratively and are not intended to limit the present invention.

The indefinite articles "a" and "an" mean "at least one" or "one or more" when used in this application, including the claims, unless specifically indicated otherwise.

The present invention is exemplified by the following comparative study. English Pointers engaged in bird hunting activities while on a Eukanuba^{®} Adult Premium Performance diet were more successful hunters, for example, recording a statistically significantly greater number of finds, and which Pointers required less food to maintain body weight, body condition, and stool consistency compared with Pointers fed either Purina^{®} Pro Plan Performance or Diamond^{®} diet. Feeding Eukanuba^{®} Adult Premium Performance diet as described and illustrated herein is more economical compared to the Purina^{®} Pro Plan or Diamond^{®} diets based on daily feeding cost or the cost per find.

The oral administration of the diet can be accomplished, for example, by mouth feeding the mammal a weight maintaining amount of the diet. The oral administration can be accomplished by mouth feeding the diet, for example, from 1 to about 10 servings per day, preferably 1 to about 2 servings per day, and more preferably 1 serving per day. The oral administration of the diet over a period of, for example, from about 1 to about 24 months or longer can produce the desired performance enhancement results and as illustrated herein. The dietary based methods for improved performance of the present invention are particularly applicable where the hunting or searching mammal is a canine, although it will be understood by one skilled in the art that the performance enhancement methods may be applicable to other hunting and working mammals. A particularly preferred canine is an English Pointer breed which is a bird-dog that has been bred, trained, or both, for game bird hunting.

In embodiments of the present invention the post-hunt rectal temperature of the canines administered the Eukanuba^{®} Adult Premium Performance Formula diet was from about 0.5 to about 1.2 degrees Fahrenheit lower compared to canines administered the Purina^{®} Pro Plan diet, and preferably the canines administered the Eukanuba^{®} Adult Premium Performance Formula diet have a post-hunt rectal temperature of from about 1.0 to about 1.1 degrees Fahrenheit lower compared to canines administered the Purina^{®} Pro Plan diet. Thus, the body temperatures of the hunting mammals fed the Eukanuba^{®} diet are maintained to within about 3.0 °F of their pre-hunt body temperatures. The oral administration in hunting canines: maintained consistent body weight during the administration period; resulted in excellent and preferred stool properties; maintained excellent overall health of the canines; maintained normal blood chemistry profiles; and maintained normal food attitudes, for example, maintained normal appetites in hunting dogs as compared to increased appetite, or more commonly, loss of appetite in high activity animals.

In embodiments the present invention provides a dietary composition administered to improve the energy utilization efficiency in an active mammal which composition comprises an effective amount of Eukanuba^{®} Adult Premium Performance Formula diet.

In embodiments the present invention provides a dietary composition administered to avoid or prevent heat exhaustion, such as overheating or heat stroke, in an active mammal which composition comprises an effective amount of Eukanuba^{®} Adult Premium Performance Formula diet.

Introduction An investigation was conducted to study the impact, if any, of diet and nutrition on canine hunt performance. Specifically, the effect of commercial diets on the quail-hunting ability of English Pointers was evaluated at a controlled-access hunting facility. A first year study compared a Eukanuba^{®} formula against a Diamond^{®} formula. A second year study compared a Eukanuba^{®} formula against a Purina^{®} formula.

Methods The working ability of English Pointers was measured in this study using a practical response criterion (hunting performance). In terms of the number of points or birds found during a hunting session, the dogs fed Eukanuba^{®} performed better than those fed either Diamond^{®} or Pro Plan^{®} diets. Although such assessments are not easily standardized in a field setting, the two handlers remained blinded to the dietary treatments during both hunting seasons. This control allowed comparisons to be made principally on the dog's hunting success during each season, when the only variation in management was the diet being fed to each group.

In the first year of this study, 23 adult Pointers were fed Eukanuba^{®} Adult Premium Performance Formula or Diamond^{®} Premium Adult Dog Food, while in year two, 22 Pointers were fed Eukanuba^{®} Adult Premium Performance Formula or Purina^{®} Pro Plan Chicken & Rice Dog Performance Formula. Dogs were initially offered amounts of food calculated to maintain body weight and body condition based on the NRC recommendation for estimating daily metabolizable energy (ME) requirements (ME = 200 BW^{0.67}), where body weight (BW) is expressed in kilograms and 200 is an activity constant for very active dogs. The handlers were instructed to adjust the food allotment of individual dogs as needed to maintain body weight and body condition during the hunting season. The decision to adjust the food allotment was at the sole discretion of the handler. Food consumption by each dog was measured by the handlers on a regular basis during the study.

Body weights and subjective stool scores were also obtained on a regular basis during the season. Subjective stool scoring was conducted using a 5-point scale: 1 = liquid; 2 = soft, no shape; 3 = soft with shape; 4 = firm (ideal); 5 = extremely dry. Body condition and skin and coat health were subjectively evaluated in September, November and March to coincide with the initiation of the training and hunting seasons, and the termination of the hunting season, respectively. The 5-point body condition scoring system consisted of: 1 = thin; 2 = underweight; 3 = ideal; 4 = overweight; 5 = obese. Skin and coat evaluations included subjective assessments of skin dander and epilation, and coat shedding, shine, uniformity, density and softness. Individuals blinded to the specific dietary assignments conducted the subjective evaluations for body condition and skin and coat health.

All statistical analyses were conducted using the GLM procedures of SAS. For statistical analysis of body weight, body condition and hunting performance data, the hunting season was divided into six 2-week hunting periods. Total times and duration of hunting, number of finds, and finds per hour were calculated on an individual dog basis for each 2-week period because all dogs were not hunted equally during each period. Despite unequal usage during the hunting season, each dog was allowed to hunt at least one time during each 2-week period. These performance data were subsequently analyzed using a statistical model that included the main effects for diet and hunting period and their interaction. Differences among treatment means were assessed by least-squares mean separation using the PDIFF^{®} option of SAS when the respective F-test for the Type III sums of squares was significant (P<0.10). For all variables, arithmetic means and their respective standard deviations are reported for each treatment group.

Principle ingredients, nutrient content and caloric distribution of each dog food are reported in Tables 1 and 2. Daily care and management of the dogs was provided by two professional handlers employed by the hunt facility. The handlers were blinded to the specific diet each dog received.

**Table 1. Major dietary ingredients of commercial diets fed to English Pointers during the quail hunting season.**

| **Eukanuba^{®1}** | **Diamond^{®2}** | **Pro Plan^{®3}** |
|---|---|---|
| Chicken | Chicken by-product meal | Chicken |
| Chicken by-product meal | Ground com | Corn gluten meal |
| Corn meal | Wheat flour | Brewers rice |
| Ground grain sorghum | Chicken fat | Beef tallow |
| Fish meal | Brewers rice | Ground com |
| Chicken fat | Beet pulp | Poultry by-product meal |
| Ground whole grain barley | Fish meal | Corn bran |
| Dried beet pulp | Egg product | Animal digest |
| Natural chicken flavor | Flaxseed | Egg product |
| Dried egg product | Poultry digest | Minerals & vitamins |
| Brewers dried yeast | Brewers dried yeast | |
| Flax meal | Minerals & vitamins | |
| Minerals & vitamins | | |
| 1. The lams Company, Daylon, OH 45414 | | |
| 2. Diamond Pet Foods, Meta, MO 65058 | | |
| 3. The Ralston Purina Company, St. Louis, MO 63164 | | |

**Table 2. Nutrient content in weight percent or caloric distribution of commercial diets fed to English Pointers during the quail hunting season.^{a}**

| **Nutrient** | **Eukanuba^{®}** | **Diamond^{®}** | **Pro Plan^{®}** |
|---|---|---|---|
| Protein (%) | 31.2 | 26.1 | 31.9 |
| Fat (%) | 21.4 | 17.2 | 21.3 |
| Crude fiber (%) | 2.1 | 3.7 | 2.0 |
| Moisture (%) | 6.7 | 8.3 | 7.7 |
| Ash (%) | 6.6 | 6.7 | 5.6 |
| Carbohydrate (%) | 31.9 | 38.0 | 31.6 |
| Calcium (%) | 1.19 | 1.50 | 1.30 |
| Phosphorus (%) | 0.97 | 1.07 | .88 |
| Ca:P | 1.23 | 1.40 | 1.48 |
| Gross energy (kcal/kg)^{b} | 5,120 | 4,660 | 5,139 |
| Metabolizable energy (ME, kcal/kg)^{b} | 4,220 | 3,899 | 4,224 |
| Protein (% of ME calories)^{b} | 28.1 | 25.4 | 28.7 |
| Fat (% of ME calories)^{b} | 43.1 | 37.6 | 42.9 |
| Carbohydrate (% of ME calories)^{b} | 28.8 | 37.0 | 28.4 |
| Total unsaturated fatty acids (%) | 14.6 | 11.9 | 13.3 |
| Total omega-3 fatty acids | 0.55 | 0.50 | 0.15 |
| omega-6 : omega-3 fatty acid ratio | 8:1 | 8:1 | 18:1 |
| ^{c}Total EPA + DHA (mean %) | 0.2440 | 0.0274 | 0.0166 |
| ^{c}EPA : DHA (mean relative ratio) | 1.052:1.137 | 0.66:1.2 | 0.37:0.44 |
| EPA+DPA+DHA (total weight percent)^{c} | 0.24 | 0.02 | 0.02 |
| Fat Source(s) (label ranking) | fish meal (5), chicken fat(6), flaxmeal(12) | chicken fat(4), fish meal (5), flaxseed(9) | beef tallow (4) |
| a) Nutrient content was determined by laboratory analyses and are expressed on an as-fed basis. | | | |
| b) Metabolizable energy content and caloric distribution were calculated using the protein, carbohydrate and fat content and the modified Atwater factors (3.8 kcal/kg; 3.8 kcal/kg; and 8.5 kcal/kg, respectively). | | | |
| c) EPA = eicosapentaenoic acid(20:5n-3); DHA1= docosahexaenoic acid (22:6n-3); DPA=docosapentaenoic acid (22:5n-3) | | | |
| 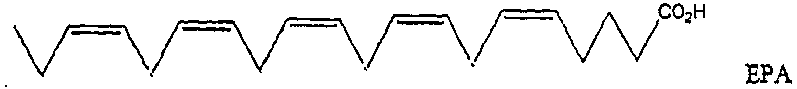 | | | |
| 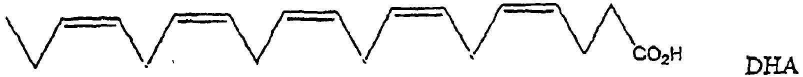 | | | |
| 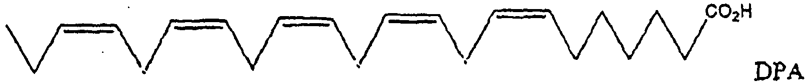 | | | |

The above mentioned comparative formulation(s) are all commercially available or may be prepared by procedures known to those in the nutritional arts. Distinguishing aspects between the above Eukanuba^{®} and the Purina^{®} Pro Plan diets follow. The Eukanuba^{®} fat content is chicken-based whereas the Purina^{®} fat content is beef-tallow based. The Eukanuba^{®} protein content is animal-based. The Purina^{®} protein is a combination of animal- and vegetable-based protein. The sources of carbohydrates and omega-3 fatty acids, levels of omega-3 fatty acids, EPA, DPA and DHA, and the ratio omega fatty acids in the Eukanuba^{®} diet are also different from the Purina^{®} diet. Distinguishing aspects between the above Eukanuba^{®} and the Diamond^{®} diets are the protein, fat, and carbohydrate amounts, the carbohydrate source, the levels of EPA, DPA and DHA, and the overall energy content of the diets and as reflected in above tables.

The source of dietary fat, the ratio of two fatty acids EPA to DHA, and the total weight percentage of two fatty acids EPA plus DHA in the Eukanuba^{®} diet are different from both the above Diamond^{®} and Purina^{®} diets and as indicated in above table. An analysis of at least seven other commercially available performance diets indicated that all had an EPA to DHA ratio (mean 1.41:1.22), and a total weight percentage of EPA plus DHA(mean 0.0374%) which were both considerably lower than the Eukanuba^{®} diet.

The above mentioned methods of the present invention can be accomplished with diets formulated with, for example, a total fat content of from about 20 to about 28 weight percent, for example, from about 20 to about 25, and from about 20 to about 23 weight percent total fat; where greater than about 75 weight percent of the total fat content is unsaturated fat, containing EPA, DHA, or a mixture of EPA and DHA, in a total amount of the diet of from 0.21 to 0.30 weight percent. In embodiments the diet can further comprise, for example, a total protein content of 30 to 35 weight percent, and a total carbohydrate content of about 30 to about 35 weight percent. In embodiments the unsaturated fat in the diet is preferably poultry fat such as chicken, fish fat, or mixtures thereof, and more preferably a combination of chicken fat, fish meal, and flax meal. The diet is preferably free of beef fat, beef protein, or both. The unsaturated fat content of the high fat-high unsaturated fat, performance diets of the present invention preferably includes a mixture of omega-6 fatty acid and omega-3 fatty acid, for example, in a weight ratio of 5:1 to 10:1, and more preferably a ratio of about 8:1, for example, as found in the above Eukanuba^{®} diet. A preferred source of omega-3 fatty acid is from, for example, fishmeal, fish oil, and flaxmeal, and more preferably fishmeal or fish oil. A preferred source of the omega-6 fatty acids is from, for example, chicken fat. A preferred type of omega-3 fatty acid is EPA, DPA, or DHA; individually or in combinations.

All dogs were subjected to the normal training and hunting program of the private hunt facility located in southwest Georgia, including training, conditioning, and hunting segments. In southwest Georgia, the quail-hunting season extends from mid-November through February and is usually preceded by a 2-month period of individual training and physical conditioning. The respective diets were fed exclusively during the training and hunting seasons. The dogs were fed once daily and they did not receive any supplements or treats. The selection of dogs for hunting and the amount of time they were allowed to hunt were based on the discretion of the handlers. The handlers recorded the dates and total time hunted for each dog, number of finds, number of flushes, general attitude of the dog, and reasons for cessation of hunting. Overall hunting performance was calculated for each dog by determining the total number of finds per hunting session and total hours hunted. Licensed veterinarians, who were also blinded to the dietary treatments, collected blood samples and conducted physical examinations at the initiation and termination of the hunting season. Serum and plasma samples were shipped to a commercial diagnostic laboratory (Antec Diagnostic, Inc. Farmingdale, NY 11735) for standard veterinary analysis.

Because performance in a warm and humid environment is an important consideration for hunting dogs in the southern portion of the United States, a temperature-humidity index (THI) was computed for each day using weather data obtained from a local television station. This index was calculated using the day's high temperature and relative humidity to provide an indication of potential heat stress. (Davenport, G.M., et al., Growth and endocrine responses of cattle to implantation of estradiol-17β during continuous or discontinuous grazing of high-and low-endophyte-infected tall fescue, J. Anim. Sci., 1993, 71:757-764.) Mild, high, and severe levels of heat stress were represented by the values 23.5 to 26, 26.0 to 29.0, and > 29.0, respectively.

**Comparative Results** The results of a two-year study showed that the nutritional management of hunting dogs can have a significant impact on their hunting performance and working ability. (Davenport, G.M., et al., Effect of Diet on Hunting Performance of English Pointers. Veterinary Therapeutics, 2001,2:10-23.) The collective results also show that nutritional management of sporting dogs can significantly impact a dog's performance and working ability. Thus, the nutritional management of canine athletes should provide a balanced diet which: is rich in nutrients required by the dog; meets energy requirements when consumed in acceptable amounts; contains optimum protein and fat levels; has a fatty acid profile that minimizes inflammation, for example, of the gut, the skin, and the like; allows for repletion (replacement) of muscle glycogen; contains the amount and type of fiber that promotes a healthy gut; is palatable and readily accepted during periods of stress, such as training, hunting or competition; is convenient to prepare and feed; and is stable at normal temperatures to avoid rancidity.

The results of the year 1 study, comparing Eukanuba with the Diamond^{®} formula, showed that all dogs remained healthy and consumed typical amounts of food throughout the hunting season. Chemical profile and complete blood count (CBC) results were within the normal ranges for adult healthy dogs and showed no major diet-induced changes in the health status of the dogs. Dogs fed Eukanuba^{®} maintained or gained weight and body condition throughout the hunting season, while dogs fed the Diamond^{®} diet lost body weight and condition (P<.05). As a result, body weight and body condition scores at the end of the hunting season were higher (P<.05) for dogs fed Eukanuba^{®} compared with the Diamond^{®} diet. No significant differences were observed in fecal stool scores despite a tendency for stools to be slightly softer for dogs fed Diamond^{®}. Dogs fed the Eukanuba^{®} formula demonstrated superior hunting performance (P<.05) compared with dogs fed the Diamond^{®} formula, based upon total finds per hunt and on the number of birds located per hour of hunting. For the season, dogs fed Eukanuba^{®} had 55% more finds than dogs fed Diamond^{®}, which was equivalent to one more find per hour of hunting. The improved hunting performance of dogs fed Eukanuba^{®} was not due to increased hunting frequency or longer hunting duration, as they were similar for both diet groups throughout the season.

During the first year (year 1 study), there were 9 days during the hunting season in which the heat index was rated as high or severe based on the temperature-humidity index. On each of these days, the dogs fed Eukanuba^{®} maintained their superior performance over Diamond^{®} based on the number of finds per hour.

In the second year (year 2 study), all dogs remained healthy based on physical examinations, chemical profiles, and CBC results. As in the year 1 study, there were no diet-induced changes in the health status of the dogs during the hunting season. Similarly, there were no differences in body weight, body condition, or stool scores during the season for dogs fed the Eukanuba^{®} formula versus the Purina^{®} Pro Plan^{®} formula. Despite these similarities, dogs fed Purina^{®} Pro Plan required 11% more food than dogs fed Eukanuba^{®} to maintain body weight and condition. This increased feeding amount was equivalent to about 2/3 cup more food per day for each dog (6.4 vs. 5.7 cups/day). There were no differences in the frequency or duration of hunting indicating that dogs on both diets had equal opportunities to hunt during this second season. The performance results showed that dogs fed Eukanuba^{®} had 33% more finds during the season than dogs fed Purina^{®} Pro Plan. Over the entire season dogs fed the Eukanuba^{®} formula averaged 3.0 finds per hunt compared with 2.2 finds per hunt for dogs fed the Purina^{®} Pro Plan formula. Weather conditions during the second year were milder than the previous year (year 1 study). As a result, dogs were not subjected to any significant heat-stressing conditions during the hunting season.

Discussion Several dietary factors may have influenced the hunting ability and body condition of the dogs used in the studies. Pointers tend to be a highly active breed and individual dogs do not typically cany additional body fat. Furthermore, these dogs typically lose a considerable amount of body condition as the hunting season progresses. Weight loss, even when moderate, is always comprised of both fat and lean body tissue. (Reinhart, G. A., Nutrition for Sporting Dogs. In: Bloomberg MS, Dee JF, Taylor RA, editors: Canine Sports Medicine and Surgery. Philadelphia, 1998, WB Saunders, pp 349; Burgess, N.S., Effect of a very-low-calorie diet on body composition and resting metabolic rate in obese men and women, J. Amer. Diet. Assoc. 1991; 91:430-434; Butterwick, F., et al. Changes in the body composition of cats during weight reduction by controlled dietary energy restriction, Vet. Rec., 1996, 138:354-357.) Unfortunately, the loss of lean tissue negatively impacts body condition and stamina which are required for sustained performance.

The caloric density of a diet may also affect the quantity of food that must be consumed to meet energy requirements. If the metabolizable energy (ME) content of the diet is too low to support increased work, the quantity of food that must be consumed may exceed the physical capacity of the digestive tract. This may lead to increased rate of passage through the digestive tract and decreased nutrient digestibility, further exacerbating an energy deficit. The production of softer stools implies that a diet may be bulk limiting which would ultimately affect energy intake, diet digestibility and nutrient availability.

Fat is a highly available energy source for the dog and its availability may affect performance during periods of strenuous work. Furthermore, it has been shown that dietary fat can affect body composition of exercising dogs, as lower fat diets result in a loss of lean tissue and body fat compared with higher fat diets that increase lean tissue and body fat. (Altom, E., Ph.D. Dissertation, *supra.)* The dog is an efficient aerobic athlete that performs best when fed a diet that supplies a large proportion of its energy as fat. (Kronfeld, D.S., Diet and the performance of racing sled dogs, J. Am. Vet. Med. Assoc., 162:470B473, 1973; Reynolds, A.J., Fuhrer, L., Dunlap, et al., Lipid metabolite responses to diet and training in sled dogs, J. Nutr., 1994, 124:2754S-2759S; Reynolds, A.J., et al., Sled dog endurance: A result of high fat diet or selective breeding? FASEB J 1995.) Controlled studies using treadmill-exercised dogs have shown that endurance is positively correlated with dietary fat intake and diet digestibility. (Altom, E., Ph.D. Dissertation, *supra;* Downey RL, et al., Diet of beagles affects stamina, J. Am. Anim. Hosp. Assoc., 1980; 16:273-277.) Furthermore, the source of dietary fat may affect hunting ability based on changes in olfactory function. Previous research has shown that olfactory sensitivity is compromised in dogs fed diets containing a greater percentage of saturated fatty acids. (Altom, E., Ph.D. Dissertation, *supra)* Therefore, inferior performance of hunting dogs could be attributed to the source of dietary fat used in a commercial formula. Thus, the use of beef tallow in commercial diets could negatively impact olfactory functionality and hunting performance compared with poultry fat and or fish fats because beef tallow contains a higher percentage of saturated fatty acids.

Effects of Fat and Heat A commonly held belief among dog trainers and breeders is that feeding a high-fat diet to working dogs can predispose them to heat stress during hot weather. However, this belief was not supported by the performance data observed during the year 1 study of the present invention. The increased fat consumed by dogs fed the Eukanuba^{®} diet compared with Diamond^{®} diet did not negatively affect the working ability or stamina of the dogs during periods of heat stress. These results are also supported by previous research showing that a reduced fat diet produced higher rectal temperatures in dogs after one hour of treadmill exercise compared with a high fat diet. (Altom, E., Ph.D. Dissertation, *supra.)* Therefore, a high fat diet may be more beneficial to a working dog during periods of hot weather based on its ability to reduce core body temperature.

Protein Dietary protein is also an important consideration for canine athletes. Current evidence indicates that aerobic training imposes an increased need for dietary protein in dogs. (Hammel, E.P., et al., Metabolic responses to exhaustive exercise in racing sled dogs fed diets containing medium, low and zero carbohydrate, Am. J. Clin. Nutr., 1976; 30:409-418; Adkins, T.O., et al., Diet of racing sled dogs affects erythrocyte depression by stress, Can. Vet. J., 1982; 23:260-263.) In all animals, athletic conditioning results in adaptive physiological changes that facilitate efficient delivery of oxygen and nutrients to working muscles. These changes include increases in blood volume, red blood cell mass, capillary density, mitochondrial volume, and in the activity and total mass of metabolic enzymes. (Kronfeld, D.S., et al., Hematological and metabolic responses to training in racing sled dogs fed diets containing medium, low, or zero carbohydrate, Am. J. Clin. Nutr., 1977; 30:419-430; Querengaesser, A., et al., Blood changes during training and racing in sled dogs, J. Nutr., 1994; 2760S-2764S.) The increased tissue mass and requirement for gluconeogenic amino acids during exercise necessitate increased protein intake by working dogs. The protein content of the diet may also impact the capacity of the blood to oxygenate tissue and transport energy-containing nutrients needed by working muscles. (Kronfeld, D.S., et al., *supra.)* Although the protein content of diets used in this study were within the range considered to be normal for healthy dogs, a slight reduction in protein content or amino acid availability may become significant during periods of increased physical activity. Thus the source of dietary protein can affect the balance and availability of amino acids that are needed by exercising tissues.

It is understood by those skilled in the art of animal feeding and diet design that diet formulas are generally not suitable for cross-feeding to other animals, for example, between dogs and cats since different animals can have family or genus specific dietary requirements or prohibitions, such as specific vitamin or mineral needs or intolerance. However, one skilled in the art recognizes these and other requirements or prohibitions and can readily reformulate basic and common nutritional components that can render them suitable for feeding to different animals. Thus the formulations of the present invention while potentially unsuitable for all animals can be readily adapted for use in other animals having similar dietary needs, physical demands, and performance objectives.

The invention will now be further illustrated by the following non-limiting Example(s). The preferred mammalian species are domesticated animals such as dogs or cat.

### Example 1

**Year 2 - Canine Rectal Temperatures** The rectal temperature of each dog was obtained by a licensed veterinarian while the dog was at rest and immediately after a 40 minute hunting session. These measurements were obtained at the end of the Year-2 hunting season (post-season collection). The body temperature of the dogs was similar before hunting regardless of diet (101.7 versus 101.4 °F). The body temperature of all dogs was higher after hunting, but the increase in body temperature was greater for dogs fed the Purina^{®} Pro Plan diet (4.5 versus 3.1 °F increase in rectal temperature). The dogs fed the Purina^{®} Pro Plan diet had significantly higher (P<0.05) body temperatures than those dogs fed the Eukanuba^{®} diet (105.9 versus 104.8 °F, a difference of 1.1). Thus the post-hunt body temperature of the dogs fed the Eukanuba diet was from about 0.5 to about 1.5 °F lower or cooler compared to the Purina^{®} Pro Plan diet. The lower or cooler post-hunt body temperature suggests the dogs expend less energy on waste heat and consequently have greater endurance and greater energy available for hunt-related tasks, such as scouting and pointing. Thus, for example, the body temperature of the hunting mammal fed Eukanuba^{®} diet is maintained to within about 3.0 °F of the pre-hunt body temperature which is significantly lower than hunting mammals fed Purina^{®} Pro Plan diet and as summarized in the accompanying Table.

### Canine Rectal Temperature (° Fahrenheit) at Post-Season Collections

| | **Eukanuba^{®}** | **Purina^{®}** | **P(<)** |
|---|---|---|---|
| **Pre-hunt** | **101.7** | **101.4** | **NS** |
| **Post-hunt** | **104.8¹** | **105.9¹** | **0.05** |
| **Difference** | **3.1** | **4.5** | |
| 1. Probability or confidence level greater than 95%. | | | |

The invention has been described with reference to various specific and preferred embodiments and techniques. However, it should be understood that many variations and modifications may be made while remaining within the scope of the claims.

## Claims

1. A non-therapeutic method selected from the group consisting of maintaining the body temperature of a hunting mammal, increasing the heat endurance of a hunting mammal, and combinations thereof, wherein the method is **characterized by** orally administering a diet which comprises a component selected from the group consisting of unsaturated fatty acid EPA, unsaturated fatty acid DHA, and mixtures thereof, wherein the diet comprises from 0.21% to 0.30% of the component by weight of the diet, wherein greater than 75% of the total fat content of the diet is unsaturated fat, by weight of the total fat content.

2. The non-therapeutic method according to any of the preceding claims wherein the diet further comprises a total fat content of from 20% to 25%, by weight of the diet.

3. The non-therapeutic method according to any of the preceding claims wherein the diet further comprises a total protein content of from 30% to 35%, by weight of the diet, and a total carbohydrate content of from 30% to 35%, by weight of the diet.

4. The non-therapeutic method according to any of the preceding claims wherein the diet comprises a component selected from the group consisting of fish meal, chicken fat, flax meal, and mixtures thereof.

5. The non-therapeutic method according to any of the preceding claims wherein the diet is free of beef fat.

6. The non-therapeutic method according to any of the preceding claims wherein the hunting mammal is a dog.

7. The non-therapeutic method according to any of the preceding claims wherein the weight ratio of EPA to DHA in the diet is from 0.8:1.5 to 1.5:0.7.

8. The non-therapeutic method according to any of the preceding claims wherein the diet further comprises the unsaturated fatty acid DPA.

9. The non-therapeutic method according to any of the preceding claims wherein the diet comprises omega-6-fatty acid and omega-3-fatty acid, wherein the weight ratio of omega-6-fatty acid to omega-3-fatty acid is about 5:1 to 10:1.

## Patentansprüche

1. Nichttherapeutisches Verfahren, ausgewählt aus der Gruppe bestehend aus dem Aufrechterhalten der Körpertemperatur eines Jagdsäugetiers, dem Erhöhen des Wärmeduldungsvermögens eines Jagdsäugetiers, und Kombinationen davon, wobei das Verfahren **dadurch gekennzeichnet ist, dass** oral eine Nahrung verabreicht wird, die eine Komponente umfasst, ausgewählt aus der Gruppe bestehend aus der ungesättigten Fettsäure EPA, der ungesättigten Fettsäure DHA und Mischungen davon, wobei die Nahrung von 0,21 Gew.-% bis 0,30 Gew.-% der Komponente umfasst, wobei mehr als 75 Gew.-% des Gesamtfettgehalts der Nahrung ungesättigtes Fett sind.

2. Nichttherapeutisches Verfahren nach einem der vorhergehenden Ansprüche, wobei die Nahrung weiterhin einen Gesamtfettgehalt von 20 Gew.-% bis 25 Gew.-% der Nahrung umfasst.

3. Nichttherapeutisches Verfahren nach einem der vorhergehenden Ansprüche, wobei die Nahrung weiterhin einen Gesamteiweißgehalt von 30 Gew.-% bis 35 Gew.-% der Nahrung und einen Gesamtkohlenhydratgehalt von 30 Gew.-% bis 35 Gew.-% der Nahrung umfasst.

4. Nichttherapeutisches Verfahren nach einem der vorhergehenden Ansprüche, wobei die Nahrung eine Komponente umfasst, ausgewählt aus der Gruppe bestehend aus Fischmehl, Hühnerfett, Leinmehl und Mischungen davon.

5. Nichttherapeutisches Verfahren nach einem der vorhergehenden Ansprüche, wobei die Nahrung frei von Rinderfett ist.

6. Nichttherapeutisches Verfahren nach einem der vorhergehenden Ansprüche, wobei das Jagdsäugetier ein Hund ist.

7. Nichttherapeutisches Verfahren nach einem der vorhergehenden Ansprüche, wobei das Gewichtsverhältnis von EPA zu DHA in der Nahrung von 0,8:1,5 bis 1,5:0,7 beträgt.

8. Nichttherapeutisches Verfahren nach einem der vorhergehenden Ansprüche, wobei die Nahrung weiterhin die ungesättigte Fettsäure DPA umfasst.

9. Nichttherapeutisches Verfahren nach einem der vorhergehenden Ansprüche, wobei die Nahrung Omega-6-Fettsäure und Omega-3-Fettsäure umfasst, wobei das Gewichtsverhältnis von Omega-6-Fettsäure zu Omega-3-Fettsäure etwa 5:1 bis 10:1 beträgt.

## Revendications

1. Procédé non thérapeutique choisi parmi le groupe constitué du maintien de la température corporelle d'un mammifère chasseur, de l'augmentation de la résistance à la chaleur d'un mammifère chasseur, et de leurs combinaisons, dans lequel le procédé est **caractérisé par** l'administration par voie orale d'un régime alimentaire qui comprend un composant choisi parmi le groupe constitué d'acide gras insaturé EPA, d'acide gras insaturé DHA, et leurs mélanges, dans lequel le régime alimentaire comprend de 0,21 % à 0,30 % du composant en poids du régime alimentaire, dans lequel plus de 75 % de la teneur totale en graisse du régime alimentaire est de la graisse insaturée, en poids de la teneur totale en graisses.

2. Procédé non thérapeutique selon l'une quelconque des revendications précédentes, dans lequel le régime alimentaire comprend en outre une teneur totale en graisse de 20 % à 25 % en poids du régime alimentaire.

3. Procédé non thérapeutique selon l'une quelconque des revendications précédentes, dans lequel le régime alimentaire comprend en outre une teneur totale en protéines de 30 % à 35 % en poids du régime alimentaire, et une teneur totale en glucides de 30 % à 35 % en poids du régime alimentaire.

4. Procédé non thérapeutique selon l'une quelconque des revendications précédentes, dans lequel le régime alimentaire comprend un composant choisi parmi le groupe constitué de farine de poisson, de graisse de poulet, de farine de lin, et leur mélanges.

5. Procédé non thérapeutique selon l'une quelconque des revendications précédentes, dans lequel le régime alimentaire est exempt de graisse de bœuf.

6. Procédé non thérapeutique selon l'une quelconque des revendications précédentes, dans lequel le mammifère chasseur est un chien.

7. Procédé non thérapeutique selon l'une quelconque des revendications précédentes, dans lequel le rapport en poids d'EPA sur le DHA dans le régime alimentaire est de 0,8:1,5 à 1,5:0,7.

8. Procédé non thérapeutique selon l'une quelconque des revendications précédentes, dans lequel le régime alimentaire comprend en outre l'acide gras insaturé DPA.

9. Procédé non thérapeutique selon l'une quelconque des revendications précédentes, dans lequel le régime alimentaire comprend de l'acide gras oméga 6 et de l'acide gras oméga 3, dans lequel le rapport en poids d'acide gras oméga 6 sur l'acide gras oméga 3 est d'environ 5:1 à 10:1.
